Europäisches Patentamt

**European Patent Office**

**Office européen des brevets**

(19)

(11) Publication number: **0 184 906**
A1

# EUROPEAN PATENT APPLICATION

(12)

(21) Application number: **85308161.0**

(22) Date of filing: **08.11.85**

(51) Int. Cl.⁴: **C 12 P 1/00, G 01 N 33/577**

(30) Priority: **09.11.84 DK 5329/84**
**19.06.85 DK 2761/85**

(43) Date of publication of application: **18.06.86**
**Bulletin 86/25**

(84) Designated Contracting States: **AT BE CH DE FR GB IT
LI LU NL SE**

(71) Applicant: **NOVO INDUSTRI A/S, Novo Allé,
DK-2880 Bagsvaerd (DK)**

(72) Inventor: **Olsson, Lennart, Ibstrupvej 20A,
DK-2820 Gentofte (DK)**

(74) Representative: **Brown, John David et al, FORRESTER &
BOEHMERT Widenmayerstrasse 4/I,
D-8000 München 22 (DE)**

(54) Monoclonal antibodies specific for human squamous lung carninoma cells, their preparation and use.

(57) A monoclonal antibody which binds an epitopic carbohydrate moiety specific for human squamous lung carcinoma, the cubstantially purified epitope and antigen containing same, as well as a method for the detection of human SLC which comprises incubating a sample containing or suspected of containing an antigen specific for human SLC with the monoclonal antibody, and detecting whether the antibody binds to the antigen.

The present invention relates generally to a diagnostic aid based on immunological principles, and more specifically to monoclonal antibodies against specific antigens produced by squamous cell lung carcinoma (SLC), to a method for making such antibodies and to their use in the diagnosis of SLC.

## Background of the Invention

Cancer cells synthesize unusual and sometimes highly specific proteins and glycolipids. Sensitive methods are needed for identifying the unusual cellular compounds with high tumor specificity, because they can provide markers for early cancer detection and perhaps for advancing understanding of the process of transformation.

This approach is needed for most forms of human cancer but especially for lung cancer which is now the main cause of cancer deaths in most countries of Western Europe and in North America. The malignant lung neoplasms can be subdivided into various histological types, of which the incidence of SLC constitutes about 50 - 60% in males.

The therapeutic possibilities for squamous lung cancer have remained very limited. One of the main obstacles seems to be that only for a minor fraction of the patients is the disease still localized at the time of diagnosis. It is therefore likely that the therapeutic efficiency using current modalities can be improved by earlier diagnosis.

0184906

Immunological procedures, and in particular those based on the use of monoclonal antibodies, are promising tools for early identification of malignant neoplasms. Both polyclonal antibodies and monoclonal antibodies with reactivity to human lung cancer cells are known in the art. Most of these antibodies react with antigens that are shared by different histological types of lung cancer and also expressed by some normal differentiated cells, in normal serum, and/or in other tumor types. A few antibodies have been reported to be specific for subtypes of lung cancer, such as small cell and large cell lung cancer (for review, see J.L. Mullshine et al., 1985. However, in these cases the specificity analysis was restricted to a limited number of cancer cell lines or a few tumor specimens, and also the biochemical characterization of the antigens has been quite limited.

So far, no report has been made of monoclonal antibodies specific for an antigen of SLC which, due to its release into the blood stream, lends itself for serological detection. Antigenic glycoproteins and glycolipids associated with various types of non-lung cancer cells are known, and carbohydrate epitopes of such glycoproteins and glycolipids have been described, see, e.g., Hakomori et al., 1985. However, no such carbohydrate epitopes of glycoproteins or glycolipids released by lung cancer cells appear to have been characterized. For a discussion of cancer-associated carbohydrate antigens detected by monoclonal antibodies, see Ginsburg, V. et al., 1984.

- 4 -                                    0184906

## Summary of the Invention

It is the object of this invention to devise a method for the detection of human SLC which in its sensitivity surpasses any of the diagnostic methods known heretofore.

The present invention is based on the observation that antigens elaborated by human SLC cell lines comprise a specific carbohydrate moiety and that such antigens are present in body fluids and tumor-containing tissue extracts from patients with squamous lung cancer.

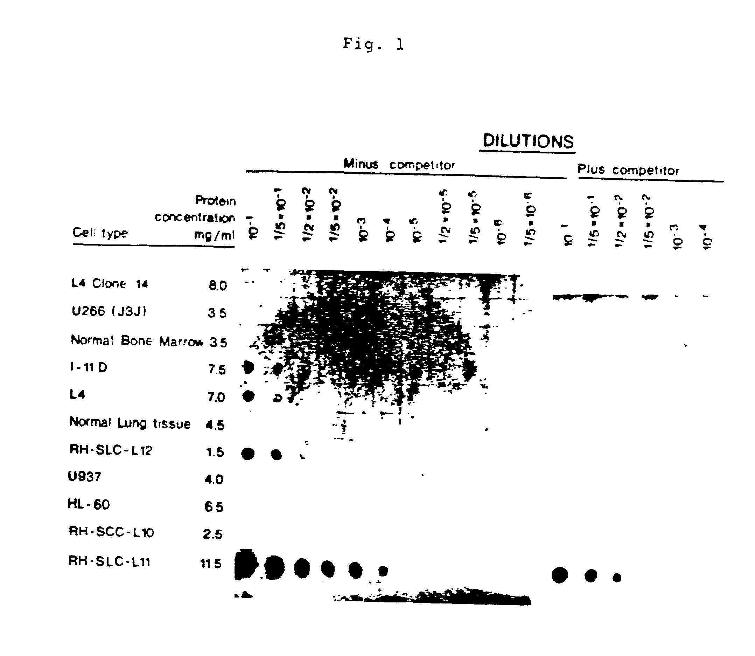

Thus, according to one of its aspects the present invention provides an SLC-associated antigen containing an epitope which is recognized by the antibody generated by the hybridoma cell line 43-9F (IgM), the latter to be defined hereinafter. Preferably, the epitope is identified as a specific carbohydrate moiety elaborated by the SLC cell line RH-SLC-Lll (cf. fig. 1 of the Drawings), or as part of an anti-idiotypic antibody which is recognized by the antibody generated by the hybridoma cell line 43-9F (IgM) or immunologically equivalent monoclonal antibodies.

According to a further aspect of the present invention there is provided a monoclonal antibody which binds an epitopic carbohydrate moiety specific for human squamous lung cancer, which moiety is produced by the cell line RH-SLC-Lll. In one preferred embodiment of the invention the monoclonal antibody is of rodent, more preferably of murine, origin. In an additional preferred embodiment the monoclonal antibody is of human origin.

The invention is also directed to a process for preparing a monoclonal antibody, which process is characterized by cultivating in a growth medium a hybridoma cell line capable of generating an antibody which binds an epitopic carbohydrate moiety specific for human squamous lung cancer, which moiety is produced by the human SLC cell line RH-SLC-L11, followed by recovery of the monoclonal antibody from the growth medium. In  preparing the monoclonal antibody of this invention, the hybridoma cell line, preferably the cell line 43-9F (IgM), is grown in tissue culture or in murine ascites culture.

Furthermore, there is provided a diagnostic kit for the detection of human SLC in a body source, which kit comprises the monoclonal antibody of Claim 5. In preferred embodiments the diagnostic kit is adapted for dot-blot analysis, Enzyme-Linked Immunosorbent Assays (ELISA) or Radio Immuno Assay (RIA).

## Brief Description of the Drawings

Fig. 1 shows dot-blot assays using the 43-9F monoclonal antibody in analysis of extracts of different cell types.

Fig. 2 shows the release of glycoproteins by RH-SLC-L11 cells.

- 6 -

0184906

Fig. 3 shows the course of exclusion chromatography on a Sephacryl® S200 column of glycoproteins released from RH-SLC-L11 cells.

Fig. 4 shows competition radioimmuno assay of the 43-9F antigen (as hereinafter defined). The assay is based on competition between immobilized antigen and antigen in solution for labelled 43-9F antibody.

## Detailed Description

The general procedures for generation of monoclonal antibodies against lung cancer cells and the methods to establish the specificity of such antibodies are known in the art (Olsson et al., 1984). A substantial number (348) of hybridomas were made by this procedure and tested for desired specificity to SLC cells. Only three of those were found to secrete immunoglobulins with a specificity of potential interest. These 3 were cloned by limiting dilution and subsequently tested for binding to 11 subclones of the RH-SLC-L11 cell line. While no binding of any of the three monoclonal antibodies to one of the 11 SLC subclones was observed they were found to react with most subclones of a small cell lung carcinoma (SCC) cell line RH-SCC-L10. Apart from these hybridomas and not included in this set of experiments one hybridoma was found to generate a monoclonal antibody with binding to all 11 subclones of the RH-SLC-L11 cell line and showing no reaction with the RH-SCC-L10 subclones. This antibody was designated 43-9F (IgM).

Additional hybridoma cell lines producing monoclonal antibodies with a similar specificity for RH-SLC-Lll cells as described above may be generated using the same procedure. In particular soluble 43-9F antigen preparations were used for immunization and screening. For further screening and identification, competitive blocking assays with the available 43-9F antibody can be used. Other rodent strains can be used; other myelomas (e.g., Taggart, R.T. et al. (1983)) can also be used.

The 43-9F (IgM) hybridoma cell line has been deposited at the National Collection of Animal Cell Cultures (NCACC), Salisbury, Wiltshire, England for the purpose of patent procedures on the indicated date.

| Deposit Date: | Depositors Reference: | NCACC Designation: |
|---|---|---|
| 14 June, 1985 | Hybridoma cell line 43-9F | 85061402 |

Dot-blot analysis or ELISA conducted according to the examples hereinafter provided was used to assay the antigen recognized by the 43-9F antibody. Fig. 1 of the drawings shows the result of an analysis of extracts prepared from several different cell types. The 43-9F antigen was readily detected in extracts of two cloned cell lines of different squamous lung carcinomas, whereas specific binding could not be detected in extracts from normal human cell types or other cancer cell lines. The 43-9F antigen - negative cell types include those derived from other types of human lung cancer cells, from normal lung cells as well as cell lines derived

- 8 -                                    0184906

from malignant neoplasms of other tissues and some other
normal tissues. Faint positive reactions were occasionally
seen with undiluted extracts of cell types other than
squamous lung carcinomas, but these reactions were attributed
to weak non-specific binding of the 43-9F antibody, since
they were not competed by excess of the 43-9F antibody,
unlike the specific reaction.

Tumor biopsies from different types of human tumors
were analysed by ELISA for the presence of antigen as summarized in Table 1. The 43-9F antibody was found to be highly
specific for squamous lung cancer, and although an occasional
isolated specimen from other tumor types did show weak
reactivity with the 43-9F antibody only squamous lung carci-
nomas were strongly positive.

**Table 1**

ELISA analysis using 43-9F antibody with cell extracts of different human malignant neoplasms.

| Tumor type | No. Specimens | No. positive[a] | | | Total pos |
|---|---|---|---|---|---|
| | | + | ++ | +++ | Total |
| **Lung:** | | | | | |
| Squamous cell carcinoma | 47 | 4 | 29 | 9 | 42/47 |
| Small cell lung cancer | 35 | 0 | 0 | 0 | 0/35 |
| Adenocarcinomas | 39 | 2 | 0 | 0 | 2/39 |
| Others | 17 | 2 | 0 | 0 | 2/17 |
| **Other carcinomas:** | | | | | |
| Stomach | 19 | 0 | 0 | 0 | 0/19 |
| Colo-rectal | 28 | 0 | 0 | 0 | 0/28 |
| Breast | 23 | 1 | 0 | 0 | 1/23 |
| Ovarian | 14 | 1 | 0 | 0 | 1/14 |
| Bladder | 19 | 1 | 0 | 0 | 1/19 |
| **Sarcomas:** | | | | | |
| Fibrosarcomas | 12 | 0 | 0 | 0 | 0/12 |
| Rhabdomyosarcoma | 1 | 0 | 0 | 0 | 0/1 |
| **Leukemias:** | | | | | |
| Acute Lymphocytic Leukemia | 11 | 0 | 0 | 0 | 0/11 |
| Acute Myeloid Leukemia | 23 | 1 | 0 | 0 | 1/23 |

[a]ELISA plates were read on automatic Titertek scanner. Background O.D. values were all 0.200. Weak reactions (+) had O.D. values of 0.2 - 0.4, strong (++) and very strong reactions (+++) had O.D. values of 0.4 - 0.8, and above 0.8, respectively.

## The 43-9F Antigen Recognized by Antibody from Hybridoma 43-9F

The dilution patterns of Fig. 1 show that the amounts of antigen produced by RH-SLC-L11 and RH-SLC-L12 cell lines are not the same. Considering the protein concentrations of the two extracts, the concentration of the antigen recognized by antibody from hybridoma 43-9F (hereinafter "antigen 43-9F") seems to be 10 - 30 fold larger in the RH-SLC-L11 cells. Fluorescence Activated Cell Sorter (FACS) analysis, immunofluorescence microscopy on permeabilized cells, and electron microscopy have all established that the 43-9F antigen primarily is a cell surface component.

The SLC cell line RH-SLC-L11 has been deposited at the National Collection of Animal Cell Cultures (NCACC), Salisbury, Wiltshire, England (NCACC) for the purpose of patent procedures on the date indicated below.

| Deposit Date: | Depositors Reference: | NCACC Designation: |
|---|---|---|
| 14 June, 1985 | RH-SLC-L11 | 85061403 |

The 43-9F antigen is released from cells in vitro. The RH-SLC-L11 cells may be grown in vitro, e.g. in RPMI-1640 medium without serum supplements, and released macromolecules binding the 43-9F antibody can therefore be readily prepared from the media.

The antigen recognized by 43-9F antibody includes either naturally occurring glycoproteins or glycolipids. This is evidenced by the isolation of substantially purified separate fractions having protein and lipid characteristics, each of which is recognized by the 43-9F antibody.

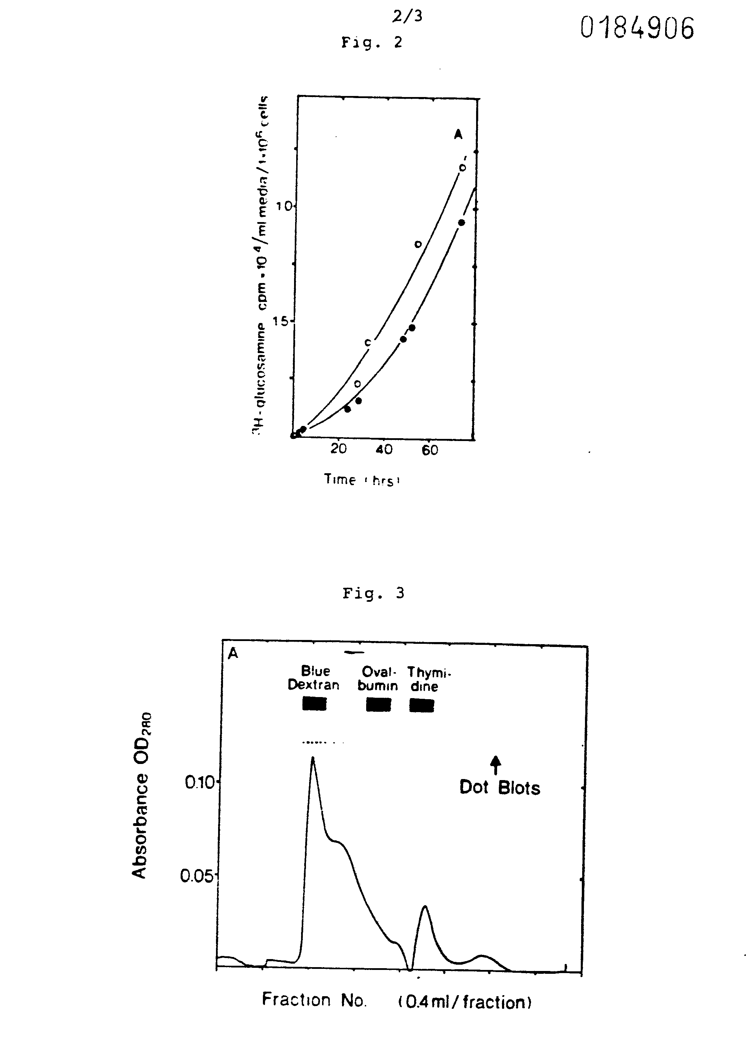

RH-SLC-L11 cells and cells from small cell lung carcinoma (SCC) (RH-SCC-L10) were cultured attached to plastic dishes in RPMI-1640 media plus fetal calf serum (FCS) ($7 \times 10^4$ cells per ml of medium). $^3$H-glucosamine (2.2 Ci/mmole, 5.0 µCi/ml) was added at zero time, 200 µl samples were removed thereafter at the indicated times, centrifuged at 30.000 r.p.m. for 10 minutes to eliminate insolubles, and TCA insoluble $^3$H-glycoprotein in the supernatants was determined. Fig. 2 of the drawings shows the release of $^3$H-glycoproteins from SLC cells (upper curve) and SCC cells (lower curve). Dot-blot analysis revealed that the 43-9F epitope is present on at least a part of the molecules released by RH-SLC cells, but not on those released by the other cell line. The rate of release of the antigen and of total $^3$H-glycoproteins was similar when the cells were grown without FCS.

The total amount of 43-9F antigen solubilized with Triton from RH-SLC-L11 cells and also that spontaneously released into media showed broad distributions of electrophoretic mobilities. Although individual bands of apparently discrete structure were seen in both distributions, no bands were clearly resolved and the positions of major bands in the adjacent immunoblot did not correspond. Coomassie blue staining of a similar gel indicated that the major stained released protein barely entered the running gel and had a molecular weight significantly greater than the 200 kD
marker. A partly resolved band at this position was also seen
in the corresponding immunoblot, but many other species of
smaller sizes having near continuous electrophoretic mobilities were also evident. To summarize, the immunoblot analysis
suggested that the molecules recognized by the 43-9F antibody
include a diverse set of primarily large glycoproteins.

Furthermore, the glycoprotein antigen has been
immunopurified using 43-9F antibody. The resulting product is
substantially free of glycoproteins which do not contain an
epitope recognized by the 43-9F antibody. This product
comprises proteins of diverse molecular weight, all of which
contain the 43-9F epitope (as hereinafter defined). This
product is part of the invention.

## The 43-9F Epitope

The term "epitope" as used in this invention is
meant to include any determinant responsible for specific
interaction with an antibody molecule. Epitopic determinants
usually consist of surface groupings of molecules such as
sugar side chains, and have specific three dimensional
structural characteristics, as well as specific charge
characteristics. The 43-9F glycoprotein and glycolipid antigen(s) will include the 43-9F epitope. However, the 43-9F
epitope may be present by itself or coupled to other than the
protein or lipid conjugates with which it is associated in nature. For example, the 43-9F epitope could be associated or coupled with proteins other than those it is normally coupled to in nature, with latex particles, and the like.

It was demonstrated by affinity chromatography that the 43-9F epitope is present on a diverse group of glycoproteins released from RH-SLC-L11 cells. A wheat germ lectin column retained nearly all of the 43-9F antigen detectable by dot-blot assay and the antigen could be eluted by 0.1 M N-acetylglucosamine. Thus, the molecules recognized by the 43-9F antibody appear to be glycoconjugates. Part of the applied antigen was also retained on a concanavalin A column and could be eluted with methyl-α-glucopyranoside, which supports the interpretation of the antigen being glycoconjugates.

The glycoproteins secreted by RH-SLC-L11 were incubated with proteinase K. Subsequent electrophoresis followed by Western blot assay showed that all reacting components in the immunoblot assay were lost. Controls incubated without the proteinase were not affected. Thus it seemed clear that the moieties recognized by 43-9F antibody are linked to proteins.

Chromatography of the glycoproteins released by RH-SLC-L11 cells on Sephacryl® S-200 showed that the bulk of the UV absorbing components as well as nearly all of the $^{3}$H-glucosamine labelled components were excluded, indicating an $M_r$ higher than 200 kD. When the glycoproteins were denatured in SDS solution and then hydrolysed exhaustively with proteinase K, $^{3}$H-labelled carbohydrate chains eluting in a molecular weight region less than that of an ovalbumin marker (45 kD) were liberated. However, part of the labelled carbohydrate chains maintained very large sizes eluting at or near the void volume.

The secreted glycoproteins were also incubated with endoglycosidase F (endo F), a glycosidase cleaving both complex and high mannose type glycans near their asparagine attachment sites in glycoproteins (Elder and Alexander, 1982). Carbohydrate chains were produced having the same size as those after hydrolysis of the glycoproteins with proteinase K, but also large carbohydrates eluting with the void volume were maintained. There was no apparent effect of the endo F on the antigenicity of 43-9F antigen in the dot-blot assay, indicating that the large glycoconjugates eluting in the void volume, resistant to hydrolysis by endo F, contain most of the epitope recognized by 43-9F antibody.

## 43-9F Antibody Recognizes a Specific Epitopic Carbohydrate Moeity

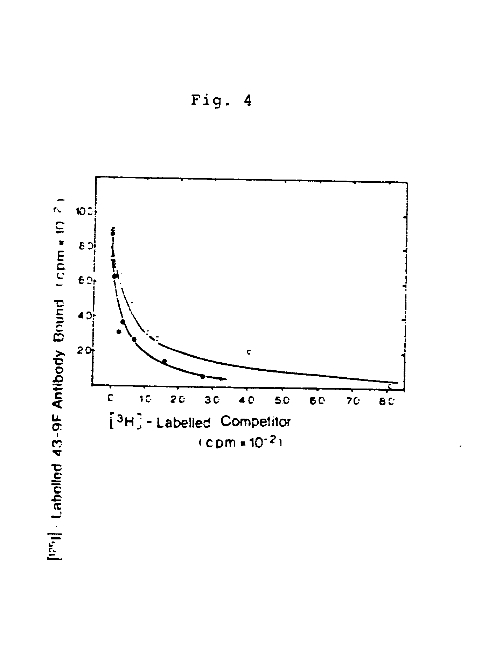

Radio Immuno Assays were established to examine the binding of $^{125}$I-labelled 43-9F antibody to 43-9F antigen in the presence of different competitors. The 43-9F antigen itself was used as competitor, as were also the carbohydrate chains purified from the specific glycoproteins after proteolysis of the associated protein as described in Example 4 hereinafter provided. All competitors were derived from the same preparation of $^3$H-glucosamine labelled glycoproteins so that the relative amounts of added competitors could be accurately compared from the $^3$H-label. As shown in Fig. 4,

$^3$H-carbohydrates eluting in the void volume of Sephacryl S200 column after exhaustive degradation with proteinase K compete even more strongly than the intact glycoproteins, suggesting that the epitope recognized by 43-9F antibody is contained in these carbohydrate chains. The enhanced competition by the large chain carbohydrates has been repeatedly observed, which rules out the possibility that the 43-9F antibody is recognizing an amino acid sequence sensitive to proteinase K. The smaller chain carbohydrates liberated after proteolysis of the glycoproteins also compete in binding the 43-9F antibody, but more weakly than the intact glycoproteins. Comparison of the competition data indicates that the concentration of the 43-9F epitope is 2.5 to 6 fold lower per $^3$H-glucosamine residue in the smaller chain carbohydrates than in those eluting in the void volume. These results seem to indicate that the epitope recognized by the 43-9F antibody is a carbohydrate sequence, specifically elaborated by the RH-SLC-L11 cells.

The 43-9F epitope could not be found on a panel of human erythrocytes, both before and after neuraminidase treatment, representing a variety of blood group antigens $(A,B,O,M,N,S,s,P_1,C,C^W,D,E,c,e,Lu^a,Lu^b,K,k,Kp^a,$ $Kp^b,Le^a,Le^b,Fy^a,Fy^b,JK^a,JK^b,Xg^a,Vel,Wr^a,Co^a,Co^b,Bu^a,I,Bg^a)$ as tested by direct and indirect agglutination and by dot-blot analysis data on solubilized membrane preparations ("white ghosts").

Glycolipid antigen was purified to homogeneity, evidenced by one spot on thin layer chromatography. This spot bound 43-9F antibody. The spot was separated. Structural analysis by methods known in the art can be carried out to define the sugar portion of the glycolipid. The isolated and purified carbohydrate portion, i.e. the isolated and pure epitope is therefore also part of this invention.

## Anti-idiotypic Monoclonal Antibodies Against the 43-9F Antibody

Balb/c mice were immunized with 43-9F antibody, for example by 3 injections over a period of 3 weeks. Mononuclear spleen cells were isolated after the last immunization and fused with the murine non-immunoglobulin-producing murine myeloma cell line X63-Ag 8.6.5.3. Hybridoma supernatants showing the ability to bind to 43-9F antibody and, in addition, to compete with 43-9F antigen in radioimmunoassay, were selected and used for the immunization of Balb/c mice. Sera from such mice were tested for binding to purified 43-9F antigen and to SLC-Lll cells. Purified 43-9F antigen substantially freed of glycoproteins which do not contain the 43-9F epitope was obtained by affinity chromatography, e.g. on a column of 43-9F antibody conjugated to Sepharose⁶.

## Detection of 43-9F Antigen in Serum

The release of large amounts of 43-9F antigen into the supernatant of RH-SLC-Lll cultures suggested that a similar process might take place in vivo, and that antigen(s) reacting with 43-9F antibody consequently could be present in the serum from SLC patients. Serum samples from 109 lung cancer patients were obtained at the time of diagnosis, and

0184906

analysed for reactivity with 43-9F antibody. In addition,
serum samples from 144 patients with other types of cancer
were analysed as well as serum from 1910 apparently healthy
individuals. As listed in Table 2 about 80% of patients with
squamous lung carcinoma were positive at the time of diagnosis, whereas the percentage of positive cases in other types
of cancers were about 6%, and in the healthy control group
about 1%.

**Table 2**

Analysis of the 43-9F antigen in serum from lung cancer patients, and from healthy persons.

| Patient Category | No. Samples (patients) | No. positive[a] | | | Total No. pos. / Total No. |
|---|---|---|---|---|---|
| | | + | ++ | +++ | |
| Lung cancer: | | | | | |
| Squamous | 76 | 3 | 49 | 8 | 60/76 |
| Small cell | 20 | 0 | 0 | 0 | 0/20 |
| Adenocarcinomas | 9 | 1 | 0 | 0 | 1/9 |
| Others | 4 | 0 | 0 | 0 | 0/4 |
| Other cancers: | | | | | |
| Gastrointestinal | 54 | 2 | 0 | 0 | 2/54 |
| Breast | 41 | 2 | 1 | 0 | 3/41 |
| Bladder | 35 | 1 | 0 | 0 | 1/35 |
| Ovarian | 32 | 1 | 2 | 0 | 3/32 |
| Sarcomas | 19 | 1 | 0 | 0 | 1/19 |
| Leukemias | 17 | 1 | 0 | 0 | 1/17 |
| Healthy Individuals: | 1910 | 18 | 1 | 0 | 19/1910 |

[a]ELISA reactions were done as described in Example 9 hereinafter.

## Discussion of the Invention

These data indicate that the majority of patients with squamous lung cancer have antigenic determinants in their serum that are highly specific for this particular type of cancer. The analysis of antigenic determinants released by cloned cell lines of squamous lung carcinoma and reacting with the same monoclonal antibody further shows that the epitope is situated on the carbohydrate part of glycoproteins mostly those having molecular weights higher than 200 kD and mucin-like or proteoglycan-like properties. Glycoproteins seem also to be synthesized and released by other cell types. The major difference peculiar to the SLC cells is a modification of the carbohydrate chains in these large glycoproteins.

The surprising feature concerning the 43-9F antigen is its specificity for squamous lung cancer. Whereas the carbohydrate epitopes of tumor associated antigens in other cancer types seem to be synthesized by several different kinds of cancer cells and also certain normal cells, no other tumor or cell type than SLC cells have been found to produce the 43-9F antigen. The analysis which involved serum samples from 250 cancer patients, 1900 serum controls, and tumor samples from nearly 300 different cancer patients has been more extensive than most prior studies of tumor associated antigens. The few instances where a non-SLC tumor scored positive in ELISA assays were primarily only weak reactions compared to SLC and the few cell types giving weak reactions in dot-blot analysis were not competed by excess antibody in the manner of the specific SLC antigen. Thus, not only does this antigen have a significantly higher specificity for human squamous lung cancer than any reported heretofore, but
it also seems to be generally one of the most specific tumor
associated antigens.

The carbohydrate sequence recognized by the 43-9F
antibody is associated with a diverse set of glycoproteins.
Those in extracts from SLC cells are primarily in mol. wt.
range of 50 to 200 kD., but one more minor component has a
mol. wt. exceeding 2000 kD. The epitope recognized by the
43-9F antibody is distinct from other known tumor-associated
carbohydrate antigens (V. Ginsburg et al., 1984).

Fig. 4 of the Drawings demonstrate that the carbohydrate chains purified from glycoconjugates shed by SLC
cells compete more strongly for binding to the 43-9F antibody
than its parent glycoconjugate.

The detection of the presence of the 43-9F complete
antigen or its characteristic epitope can be carried out in
sources or samples such as serum, blood, saliva, urine,
tissue, biopsies, bronchial lavage and the like.

The detection can be by immunoassay, such as by
competitive or immunometric ("sandwich") types. In a competitive assay, the purified 43-9F antigen, or molecule
comprising its characteristic epitope, is labelled with a
detectable label. The sample suspected of containing the 43-
9F antibody or epitope is incubated with 43-9F antibody and
labelled 43-9F antigen, and after formation of any immune
complexes, separation and detection, the level of 43-9F
antigen is readily determined.

- 21 -

0184906

Another type of competitive assay involves immobilized 43-9F antigen or epitope and labelled 43-9F antibody. The presence of 43-9F antigen in the source or sample prevents binding of antibody to immobilized antigen or epitope, thus providing an inverse measure of the presence of antigen.

In a competitive type assay, the antibody may also be immobilized on a solid phase, either previous to or subsequent to the formation of the immune complex. For example, a second, anti-mouse IgG, antibody immobilized on a solid phase can be added to the mixture, in what is known as a "double antibody" technique.

In an immunometric (sandwich) assay, one sample of the 43-9F antibody is detectably labelled. Another sample of the antibody is insolubilized on a solid phase. Incubation of sample with labelled and insolubilized antibody leads to a sandwich, where, after separation of unbound antibody(ies) the amount of label is proportional to the amount of antigen. Immunometric assays can be carried out in forward, reverse or simultaneous modes, depending on the order of addition of the insolubilized and/or labelled antibodies.

For increased sensitivity in the sandwich system, the procedures described can be modified using biotinylated 43-9F antibody reacting with avidin-peroxidase conjugates.

Instead of the 43-9F antigen or characteristic epitope, it is possible to use, in assays, anti-idiotypic antibodies or immunologically active fragments thereof produced by raising antibodies against the binding site of monoclonal antibody 43-9F.

The materials for use in the assay of the invention are ideally suited for the preparation of a kit. Such a kit may comprise a carrier means being compartmentalized to receive in close confinement one or more container means such as vials, tubes, and the like, each of said container means comprising one of the separate elements to be used in any desired method.

For example, one of the said container means useful in immunometric assays may comprise monoclonal antibody 43-9F bound to a carrier. A second container may comprise soluble, detectably labelled monoclonal antibody 43-9F, in lyophilized form or in solution.

A kit useful for a competitive assay would comprise a first container comprising detectably labelled 43-9F antigen, and a second container comprising 43-9F antibody, free or carrier-bound.

In addition, the carrier means may also contain a plurality of containers each of which comprises different, predetermined amounts of 43-9F antigen. These latter containers can then be used to prepare a standard curve into which can be interpolated the results obtained from the sample containing the unknown amount of antigen.

Practice of the present invention will now be
further illustrated by way of the following specific examples
which, however, are not to be construed as limiting the scope
of the invention.

## Example 1

## Tumor Specimens and Cell Cultures

Tumor specimens were obtained as fresh biopsy
material from patients with a variety of malignant diseases.
Institutions contributing specimens were: State University
Hospital, Copenhagen; Aalborg Sygehus, Aalborg, Denmark;
Department of Clinical Chemistry, Oslo, Norway; Institute of
Cancerology and Immunogenetics, Paris, France; Stanford
University School of Medicine, Stanford, California; City of
Hope Medical Center, Duarte, California. The indicated diag-
noses were those of the contributing institution. Most of the
material was homogenized after isolation of the cortical part
of the tumor that normally contains most of the tumor cells,
frozen in RPMI-1640 containing 30% FCS with 10% DMSO, and
stored in liquid nitrogen. The leukemia samples were obtained
from fresh marrow aspirates. Serum samples from cancer
patients were obtained at the time of diagnosis at the institutions just named. Serum samples from healthy persons were
obtained from blood donors at the following blood banks:
State University Hospital, Copenhagen, Denmark; Department of
Clinical Chemistry, Oslo, Norway; and Hôpital Paul Brousse,
Paris, France. The human SLC cell lines RH-SLC-L11 and RH-
SLC-L12 were cultivated in RPMI-1640 medium plus 15% fetal
calf serum (RPMI/FCS) as previously described (Olsson et al.,
1984). The cells are routinely grown attached in plastic vessels. They can also grow in serum-free RPMI-1640 medium for periods up to 7 days with growth rates only slightly less than that in serum containing media. A human small cell lung carcinoma line RH-SCC-L10 was similarly maintained as described (idem, ibid.). The human cancer cell lines described in Fig. 1 were also grown in RPMI/FCS as were also the promyelocytic HL-60 leukemia (Gallagher et al. 1979), U937 histiocytic lymphoma (Sundström and Nilsson, 1976), RH-L4 B-lymphoma (Olsson et al., 1983), and foreskin fibroblasts. Normal human mononuclear leucocytes and erythrocytes were isolated as described previously (idem, ibid.). Normal human lung cell populations were obtained from patients that underwent lobectomy of the lung for reasons other than malignant disease. The biopsies for analysis included a piece of bronchus.

## Example 2

## Purificaton of Glycoproteins Released by SLC Cells

The RH-SLC-L11 cells were grown in 150 $cm^2$ or 300 $cm^2$ plastic dishes at 37°C in 5% $CO_2$ atmosphere in RPMI-1640 media minus FSC at cell densities at least $2 \times 10^4$ cells per ml . After 4 - 5 days of growth, the medium was withdrawn, chilled to 4°C and centrifuged at 30,000 g for 20 minutes to remove cell debris or other insoluble material. A solution of saturated ammonium sulfate (Sigma Grade VI) was added slowly with continuous mixing to provide a 75% w/v final concentration, and stirred overnight at 4°C. The amount of medium processed at a single time varied from 100 ml to 1.5 l. Precipitates were collected by centrifuging at 30,000 g for 30 minutes and resuspended in a small volume of 0.1 M sodium phosphate pH = 7.0 at a protein concentration of about 2 to 5 mg per ml. Protein concentrations were estimated from UV absorption assuming that a solution of 1.0 mg per ml has $O.D._{280}$ = 1.0. The preparation or aliquots thereof were then applied to Sephacryl S200, S300, or S400 columns (Pharmacia) (25 cm x 0.8 $cm^2$) equilibrated with PBS or with PBS plus 0.1% Tween and the column was eluted with the same solution at a flow rate of 20 ml per hr. As shown in Fig. 3, the bulk of the glycoproteins and 43-9F antigen eluted with the void volume. Recovery of glycoprotein was nearly 100% when Tween was included in solutions but was usually less than 30% of applied material without Tween. After degradation with proteinase K, carbohydrates were quantitatively recovered with or without Tween.

## Example 3

## $^3$H-labelled Glycoproteins

To prepare $^3$H-labelled glycoproteins, the RH-SLC- L11 cells were grown for the last 2 days in serum-free RPMI- 1640 media in the presence of $^3$H-glucosamine (Amersham) 3- 10μCi/ml, 3.5 Ci/mmole. The media was collected and labelled glycoproteins purified as just described.

## Example 4

## Purification of Carbohydrates from Glycoproteins

The glycoproteins released by RH-SLC-L11 cells, purified as just described, were boiled for 2 minutes in a solution containing 0.10 M sodium phosphate of pH 7.0 plus 0.10% SDS and then chilled. The solution was diluted 4-fold with the same phosphate buffer to give a protein concentration of 200 - 300 µg per ml and proteinase K (Boehringer Mannheim), autodigested for 2 hours at room temperature was added to a final concentration of 375µg per ml. This was incubated for 16 - 18 hours at 37°C, additional proteinase K was added to a final concentration of 750 µg per ml and incubation continued for 2 - 3 hours. The solution was boiled for 10 minutes, cooled and applied to a Sephacryl S200 column to separate smaller chain carbohydrates from those eluting at or near the void volume.

## Example 5

### Antibody 43-9F (IgM)

The antibody was secreted by the deposited mouse hybridoma cell line. Ascites fluid was partially purified by passing through a 100 cm x 30 cm$^2$ S300 Sephacryl column and collecting fractions from the void volume. The most highly purified antibody was prepared from supernatant obtained from serum-free HB102 media (Hana Biologicals, California) in which the 43-9F hybridoma was grown. The IgM was precipitated from the media with 50% w/v ammonium sulfate and applied to Sephacryl S300 column equilibrated with PBS. Fractions from the void volume contained IgM contaminated with only trace amounts (less than 0.5%) of two other proteins detectable by Coomasie blue staining after electrophoresis on 10% polyacrylamide gels.

## Example 6

## Labelling with $^{125}$I

        Glycoproteins and antibodies were labelled either via lactoperoxidase coupled to beads (Biorad) or using the IodoGen (Pierce) reagent (Fraker and Speck, 1978). Labelled proteins were separated from free iodine by centrifugation through a small S25 Sephadex column mounted in a centrifuge tube to catch the flow-through containing labelled proteins. Specific activity of antibodies used here was generally in the range of 0.5 - 2 x $10^6$ cpm per µg to avoid multiple labelled molecules. The amount of labelled antibody able to recognize antigen was estimated by applying the antibody to an affinity column (Affigel-Biorad) in which total proteins in a Triton lysate of RH-SLC-L11 cells were conjugated.

## Example 7

## Dot-blot Analysis

        Cell extracts or purified proteins were analysed for binding of antibodies in a dot-blot assay with antigen attached to nitrocellulose sheets (Biorad). Cultured cancer or normal cells were collected from their growth media by centrifugation, washed in PBS and then washed and resuspended in a solution containing 0.02 M Tris, pH 7.5, 0.02 M NaCl, 0.2 mM PMSF, 0.10 M sucrose at a concentration of 1 x $10^7$ to 1 x $10^8$ cells per ml (depending on cell volume). The RH-SLC-L11 cells were suspended at 2 x $10^7$ cells/ml. These cells had been scraped loose from plastic dishes with a rubber policeman. Cells were disrupted in the presence of 1.0% Triton X-

0184906

100 in a Potter-Elverhjem homogenizer and soluble proteins separated from nuclei and cell debris, all as described (Lydersen and Pettijohn, 1980). Protein concentration in the cell-free extract was determined in an acidic Coomasie brilliant blue solution as described (Sedmak and Grossberg, 1977). Crude extracts or partially purified proteins were applied to nitrocellulose by absorbing a measured sample of 2 - 5 μl directly with a micropitette and allowing the sample to air dry. The sheet was then washed in an agitated tray with 2 changes of PBS solution containing 0.05% Tween. Some of the applied protein usually washed off at this time. Measured from retained $^3$H-radioactivity, the $^3$H-glycoproteins purified from media were irreversibly bound to an extent of 20 - 25%, when applied as described at a concentration of approximately 500 μg per ml or less. The sheets were then incubated in sealed plastic bags with $^{125}$I-labelled antibody (1 - 2 x 10$^6$ cpm per 100 cm$^2$ nitrocellulose) in PBS solution containing 30% FCS plus 0.05% Tween. After incubation for 2 hours at room temperature in a shaker, the nitrocellulose was removed and washed in PBS-Tween solution over a 6 - 12 hours period with 4 - 5 changes; the sheet was then dried and applied in a plastic envelope (to eliminate possibility of $^3$H-radioactivity exposing film) to Kodak X-ray film at -60°C to obtain an autoradiogram of the dot-blots. To estimate the relative amount of $^{125}$I-labelled antibody bound in dot-blots, autoradiograms were scanned with a Zeiss recording micro-densitometer and integrated via a Brock and Michelsen digital processor. Calibrations were made by scanning autoradiograms of known amounts of $^{125}$I-monoclonal antibody. These showed that a linear relationship between integrated areas and bound monoclonal antibody existed over a limited range.

## Example 8

### Solid Phase Radioimmuno Assay

RH-SLC-Lll released glycoproteins, purified as described in Example 2 were absorbed to the surface of plastic microtiter wells for radioimmune assay. After blocking non-specific absorbing sites, each well was incubated for 4 hours with $^{125}$I-labelled 43-9F antibody in 50 µl of PBS containing 1% of FCS. Variable amounts of purified $^{3}$H-glucosamine labelled RH-SLC-Lll released glycoproteins or carbohydrates purified from the same labelled glycoproteins were added as competitors with the labelled antibody. After incubation and washing, the bound $^{125}$I-labelled antibody in each well was counted in a gamma counter. The results are shown in Fig. 4. Each data point is an average of 3 identical analyses. Upper curve: Competitor was intact $^{3}$H-glycoproteins; lower curve: Competitor was carbohydrate chains purified on Sephacryl columns as described in Example 4.

## Example 9

### Enzyme-linked Immunosorbent Assay (ELISA)

For testing reactivity of tumor cells with the 43-9F antibody by ELISA methods Triton X-100 extracts were prepared from tumors and wells of 96 well plates coated with extract corresponding to roughly $10^5$ - $10^6$ cells, all as described above. The monoclonal 43-9F antibody was peroxidase conjugated and the ELISA assay therefore carried out as one-step procedure. The plates were incubated with the specific antibody (ca. 100 ng per well) for 1 hour at 20°C and 1 hour at 37°C, washed in PBS/Tween, and incubated with substrate as described by Kennett (1980) and read after 15 minutes and ´5 minutes on an automatic Titertek scanner. The values at 45 minutes were used. Optical density (O.D.) for background values were below 0.200, weak (+) reactions were O.D. 0.2 - 0.4, strong (++) and very strong (+++) reactions had O.D. values of 0.4 - 0.8 and higher than 0.8, respectively. Some analyses were done in parallel with the 43-9F monoclonal antibody both as catching and detecting antibody. However, the latter type of ELISA was not superior to the one-step procedure described above. In contrast, this sandwich ELISA assay was used for analysis of antigen in serum. The one-step procedure could not be used in this case, because the serum proteins compete for attachment of the specific antigens to plastic. The assay was therefore done as sandwich assay in which the plates coated with unlabelled 43-9F antibody (1 µg per ml, 100 ng per well), washed in PBS/Tween, blocked with PBS/1% BSA, incubated with serum diluted 1:1 in PBS for 1 hour at room temperature and 1 hour at 37°C, washed, and incubated for 2 hours at room temperature with peroxidase - conjugated 43-9F. The reading of the test was done as described above.

For increased sensitivity in the ELISA system, the procedures described can be modified using biotinylated 43-9F antibody reacting with avidin-peroxidase conjugates.

0184906

## REFERENCES

Elder, J.H., and Alexander, S. (1982). Endo- β -N-Acetylglu-cosaminidase F. Endoglycosidase from Flavobacterium Meningo-septicum that cleaves both high mannose and complex glycoproteins. Proc. Natl. Acad. Sci USA 79, 4540 - 4544.

Fraker, P.J., and Speck, J.C. Jr. (1978). Protein and cell membrane iodinations with a sparingly soluble chloramide 1,3,4,6-tetrachloro-3a,6a-diphrenylglycoluril. Biochem. Biophys. Res. Commun. 80, 849 - 857.

Gallagher, R., Collins, S., Trujillo, J., McCredie, K., Ahearn, M., Tsai, S., Metzgar, R., Aulakh, G., Ting, R., Ruscetti, F., and Gallo, R.C. 1979). Characterization of the continuous differentiating myeloid cell line (HL-60) from a patient with acute promyelocytic leukemia. Blood 54, 713 - 733.

Ginsburg, V., Fredman, P., and Magnani, J.L. (1984). Cancer-associated carbohydrate antigens detected by monoclonal antibodies. in "Genes and Antigens in Cancer Cells - The Monoclonal Antibody Approach"; Contributions to Oncology, Vol. 19, 51 - 63. G. Riethmuller, H. Koprowski, S. von Kleist, and K. Munk, eds. (Karger, Basel).

Hakomori et al., Can.Res. 45, 2405 - 2414 (1985).

Kennett, R.H. (1980). Enzyme-linked antibody assay with cells attached to polyvinyl chloride plates. In Monoclonal Antibodies, R.H. Kennett, J. McKearn, and K.B. Bechtol eds. (Plenum Press, New York) 376 - 377.

Lydersen, B.K. and Pettijohn, D. (1980). Human-specific nuclear protein that associates with the polar region of the mitotic apparatus: Distribution in a human/hamster hybrid cell. Cell 22, 489 - 499.

Mullshine, J.L. et al. in: Monoclonal Antibodies in Cancer pp. 229 - 246 (Sell, S. and Reisfeld, R.A., eds.) Humana Press, N.J., USA, (1985).

Olsson, L., Kronstrøm, H., Cambon-de-Mouzon, A., Honsik, C.J., Brodin, T., and Jacobsen, B. (1983). Antibody-producing human-human hybridomas. I. Technical aspects. J. Immunol. Methods 61, 17 - 32.

Olsson, L., Sørensen, H.R., and Behnke, O. (1984). Intratumoral phenotypic diversity of cloned human lung tumor cell lines and consequences for analysis with monoclonal antibodies. Cancer 54, (1757 - 1765).

Sedmak, J.J. and Grossberg, S.E. (1977). A rapid, sensitive, and versatile assay for protein using coomasie brilliant blue G250. Anal. Biochem. 79, 544 - 552.

0184906

Sundström, C., and Nilsson, K. (1976). Establishment and characterization of a human histiocytic lymphoma cell line (U-937). int. J. Cancer 17, 565 - 577, 1976.

Taggart, R.T. et al. Science 219 1228 (1983).

The features disclosed in the foregoing description, in the following claims and/or in the accompanying drawings may, both separately and in any combination thereof, be material for realising the invention in diverse forms thereof.

0184906

CLAIMS


1.      An antigen containing an epitope which is
recognized by the antibody produced by the hybridoma cell
line 43-9F (IgM) or immunologically equivalent monoclonal
antibodies.


2.      The antigen of Claim 1, wherein the epitope is a
specific carbohydrate moiety produced by the squamous lung
carcinoma cell line RH-SLC-L11.


3.      The antigen of Claim 1, wherein the epitope is an
anti-idiotype antibody moiety or an immunological equivalent
thereof.


4.      A substantially purified carbohydrate moiety
capable of binding specifically to the antibody produced by
hybridoma 43-9F.


5.      A monoclonal antibody for use as a diagnostic aid
for the detection of lung cancer, characterized by binding an
epitopic carbohydrate moiety specific for human squamous lung
carcinoma, which moiety is produced by the cell line
RH-SLC-L11.


6.      The monoclonal antibody of Claim 5, characterized
by being of rodent or human origin.

7.      The monoclonal antibody of Claim 5, characterized in that it is obtainable from the hybridoma cell line 43-9F (IgM) or clones or subclones thereof.

8.      A hybridoma secreting an antibody which binds to an epitopic carbohydrate moiety specific for human squamous lung carcinoma, which moiety is produced by cell line RH-SLC-Lll.

9.      A process for preparing a monoclonal antibody for use as a diagnostic aid for the detection of lung cancer, which process is characterized by cultivating in a growth medium a hybridoma cell line capable of generating an antibody which binds an epitopic carbohydrate moiety specific for human squamous lung carcinoma, which moiety is produced by the cell line RH-SLC-Lll.

10.     The process of Claim 9, wherein the hybridoma cell line is the cell line 43-9F (IgM).

11.     A method for the detection of human squamous lung carcinoma (SLC) which comprises contacting a source containing or possibly containing an antigen specific for human SLC with the monoclonal antibody of Claim 5, 6 or 7 and detecting whether said antibody binds to said antigen.

12.     A diagnostic kit for the detection in serum, in tissue or tissue extracts of human SLC comprising the monoclonal antibody of Claim 5.

13.     The diagnostic kit of Claim 12, adapted for dot-blot analysis, an immunometric sandwich assay or a competitive assay.

14.        The diagnostic kit of Claim 12 adapted for immuno-
histochemical localization of human squamous lung carcinoma
cells.

15.        A diagnostic kit comprising a carrier being
compartmentalized to receive in close confinement therein two
or more containers,
           a first container comprising an antigen containing
an epitope recognized by the antibody produced by hybridoma
43-9F, and
           a second container comprising a detectably
labelled, monoclonal antibody which binds an epitopic carbohydrate moiety specific for SLC, which moiety is produced by
the cell line RH-SLC-L11.

16.        The kit of Claim 15 wherein said antibody in said
second container is produced by the hybridoma cell line
43-9F.

Fig. 1

Fig. 2

Fig. 3

Fig. 4

[¹²⁵I] - Labelled 43-9F Antibody Bound (cpm × 10⁻²)

[³H] - Labelled Competitor
(cpm × 10⁻²)

0184906

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | CANCER, vol. 59, no. 9, 1st November 1984, pages 1757-1765, Philadelphia, US; L. OLSSON et al.: "Intratumoral phenotypic diversity of cloned human lung tumor cell lines and consequences for analyses with monoclonal antibodies" * Page 1757, paragraph 3; page 1760, last subtitle - page 1765 * | 1-10 | C 12 P 1/00 G 01 N 33/577 |
| A | WO-A-8 201 072 (MASSACHUSETTS GENERAL HOSPITAL) * Claims 1,7,8,15,17 * | 1-10 | |
|  | | | **TECHNICAL FIELDS SEARCHED (Int. Cl.4)** |
|  | | | C 12 P C 12 N G 01 N |

The present search report has been drawn up for all claims

| Place of search | Date of completion of the search | Examiner |
|---|---|---|
| THE HAGUE | 24-01-1986 | CUENDET P.A. |